# EUROPEAN PATENT APPLICATION

(11) **EP 4 830 594 A1**
(43) Date of publication of application: **28.10.2026**
(21) Application number: 26158179.7
(22) Date of filing: 12.02.2026
(51) Int. Cl.: A47G 9/00, A47G 9/10

(54) **MOXIBUSTION THERAPY PILLOW**

(30) Priority: 24.04.2025 CN 202520796945 U
(71) Applicant: Guangzhou New Design Biotechnology Co., Ltd, Guangzhou, Guangdong 510220 (CN)
(72) Inventor: ZHANG, Shaowei, Guangzhou, 510220 (CN); ZOU, Sile, Guangzhou, 510220 (CN); LIN, Jiehong, Guangzhou, 510220 (CN); OU, Yuhui, Guangzhou, 510220 (CN)
(74) Representative: Patent 42

(57) **Abstract**

Provided is a moxibustion therapy pillow including a pillow ring arm and a moxibustion therapy module surrounded by the pillow ring arm, where the moxibustion therapy module includes a breathable panel, a heating chamber structure, and an insertion piece, the breathable panel is provided with an insertion hole, the heating chamber structure includes a chamber cover and an electric conductive heating element installed in the chamber cover, a chamber opening end of the chamber cover is connected to a bottom surface of the breathable panel, the insertion piece is placeably and removably installed in the insertion hole for delivering a moxa sheet to the electric conductive heating element for heating, and moxa heat emitted from the heated moxa sheet is dispersed through the breathable panel. The moxibustion therapy pillow provided by the present disclosure not only serves as a pillow but also integrates a moxibustion therapy function.

## Description

### TECHNICAL FIELD

The present disclosure relates to the technical field of physiotherapy devices, and in particular to a moxibustion therapy pillow.

### BACKGROUND

Pillows, cushions, etc., placed on sofas, beds, automobile seats, etc. to provide support and improve comfort, are items commonly used in daily life, and these items also help alleviate fatigue. The pillows generally have a single function for support. At present, there are also some pillows with additional functions, such as incorporating Chinese herbal medicines or tea stalks inside, which have certain medicinal effects or fragrance. Some can also be used for moxibustion by incorporating moxibustion devices, such as the commonly seen moxibustion pillows in the industry. However, most of the current moxibustion pillows are still used as pillows and even adopt open flame moxibustion, which poses certain safety hazards. The pillows integrating a moxibustion function are rarely available on the market, as there is difficulty in inserting and removing moxibustion sheets and it is also related with stability of heat absorption due to the need for the pillows to be inserted upright or randomly.

### SUMMARY

Provided is a moxibustion therapy pillow that improves safety in use, is convenient to insert and remove moxa sheets, and provides stable heat absorption.

A technical solution adopted by the present disclosure is a moxibustion therapy pillow including a pillow ring arm and a moxibustion therapy module surrounded by the pillow ring arm, where the moxibustion therapy module includes a breathable panel, a heating chamber structure, and an insertion piece, the breathable panel is provided with an insertion hole, the heating chamber structure includes a chamber cover and an electric conductive heating element installed in the chamber cover, a chamber opening end of the chamber cover is connected to a bottom surface of the breathable panel, the insertion piece is placeably and removably installed in the insertion hole for delivering a moxa sheet to the electric conductive heating element for heating, and moxa heat emitted from the heated moxa sheet is dispersed through the breathable panel.

Further, the moxibustion therapy module includes a fan, an air intake is provided on a bottom wall of the chamber cover, the fan is mounted at a position of the air intake to blow air into the chamber cover, so that the moxa heat is dispersed through the breathable panel.

Further, the heating chamber structure includes a heat insulating column, and the heat insulating column is mounted in the chamber cover to support the conductive heating element.

Further, the moxibustion therapy module includes a bottom cover, the bottom cover being connected to the bottom surface of the breathable panel and covering the chamber cover, and the fan is mounted inside the bottom cover.

Further, an air intake duct is provided on the pillow ring arm to communicate with the interior of the bottom cover.

Further, a power supply and a circuit board are mounted inside the bottom cover.

Further, the breathable panel includes a panel part, on which a light transmitting hole is formed, and the light transmitting hole is used in conjunction with a phototherapy device to allow light to pass through.

Further, the insertion member includes a sealing and blocking plate part and heat-resistant prongs formed at a bottom end of the sealing and blocking plate part, the sealing and blocking plate part being fitted with the insertion hole for fixation, and the heat-resistant prongs are configured to insert and remove the moxa sheet.

Further, a pressure conduction part is provided on the sealing and blocking plate part to conduct the heating circuit of the conductive heating element when the insertion member is inserted.

Further, the moxibustion therapy pillow includes an elastic band, both ends of which being connected to the pillow ring arm for elastically binding the moxibustion therapy pillow to an external object.

Further, the moxibustion therapy pillow includes a controller, and a cloth bag is formed on the pillow ring arm to accommodate the controller.

Compared with the prior art, the moxibustion pillow of the present disclosure heats the moxa sheet by using a conductive heating element, obviating the need for open flame moxibustion and ensuring safety in use. The moxa sheet is inserted and removed by the insertion member and heated in the heating chamber structure, and the moxa heat is dispersed through the grid holes of the breathable panel. The moxa sheet can be conveniently loaded and unloaded, and achieves stable heating. At the same time, it achieves large-area dispersion of moxa heat, facilitating large-area moxibustion therapy on local areas of the body. The moxibustion pillow is convenient for daily use. Moreover, through the structural design of the chamber cover, the dispersed moxa heat can be evenly heated on the surface of a user.

### BRIEF DESCRIPTION OF THE DRAWINGS

The accompanying drawings are provided for a further understanding of the present disclosure, constitute a part of the specification, and together with the detailed embodiments set forth below, explain the present disclosure, but shall not constitute a limitation thereon. In the drawings:
Fig. 1 is a perspective view of a moxibustion therapy pillow according to the present disclosure;
Fig. 2 is a first exploded view of a moxibustion therapy pillow according to the present disclosure;
Fig. 3 is a front view of a moxibustion therapy pillow according to the present disclosure;
Fig. 4 is a cross-sectional view of a moxibustion therapy pillow according to the present disclosure;
Fig. 5 is a second exploded view of a moxibustion therapy pillow according to the present disclosure;
Fig. 6 is an exploded view of a moxibustion heat component of a moxibustion therapy pillow according to the present disclosure;
Fig. 7 is a view from another perspective of a moxibustion heat component as shown in Fig. 5;
Fig. 8 is a perspective view of a heat dissipation chamber of a moxibustion therapy pillow according to the present disclosure;
Fig. 9 is a back view of a moxibustion therapy pillow according to the present disclosure; and
Fig. 10 is a top view of a moxa sheet used in a moxibustion therapy pillow according to the present disclosure.

There are the reference numerals:
pillow ring arm 10; moxibustion therapy module 20; breathable panel 21; heating chamber structure 22; insertion member 23; grid hole 211; insertion hole 212; chamber cover 221; conductive heating element 222; air intake 2211; heat insulating column 223; heating cavity 2221; fan 24; bottom cover 25; fan frame 26; boss part 261; screw sleeve 262; fan mounting hole 263; mounting hole 11; step groove 111; air intake duct 12; filter sheet 13; light transmitting hole 213; base plate 201; heating element 202; chamber tube 203; positioning column 204; sealing and blocking plate part 231; heat-resistant prong 232; clasp 2311; snap ring 233; snap groove 2331; insertion holding hole 51; pressure conduction part 234; conductive spring 2332; handle cover 235; elastic band 41.

### DETAILED DESCRIPTION OF THE EMBODIMENTS

Hereinafter, the detailed embodiments of the present disclosure will be described in detail in conjunction with the accompanying drawings. It should be understood that the detailed embodiments described herein are intended solely for the purpose of illustrating and explaining the present disclosure, and are not intended to limit the same.

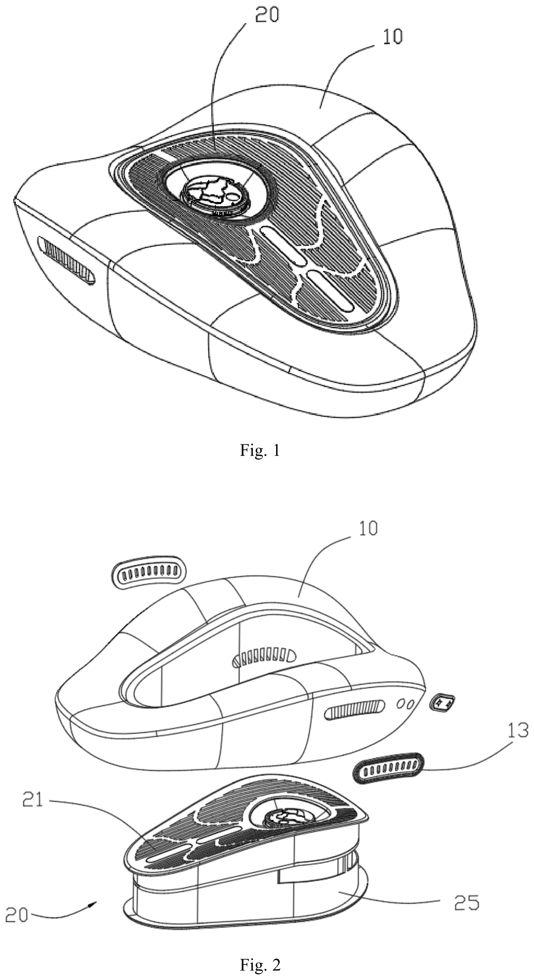

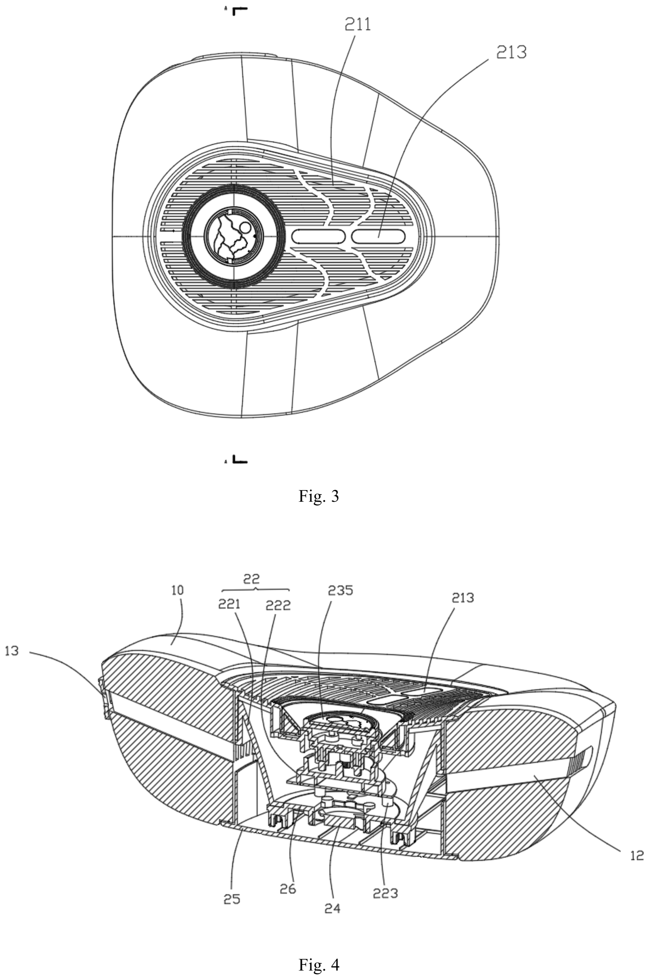

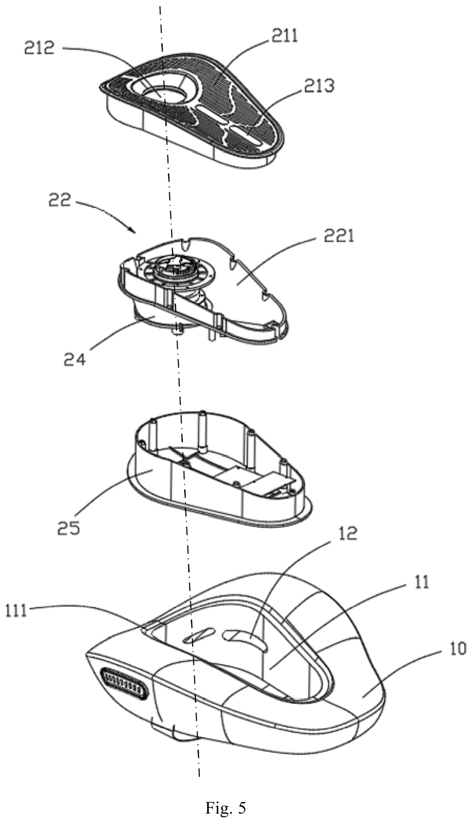

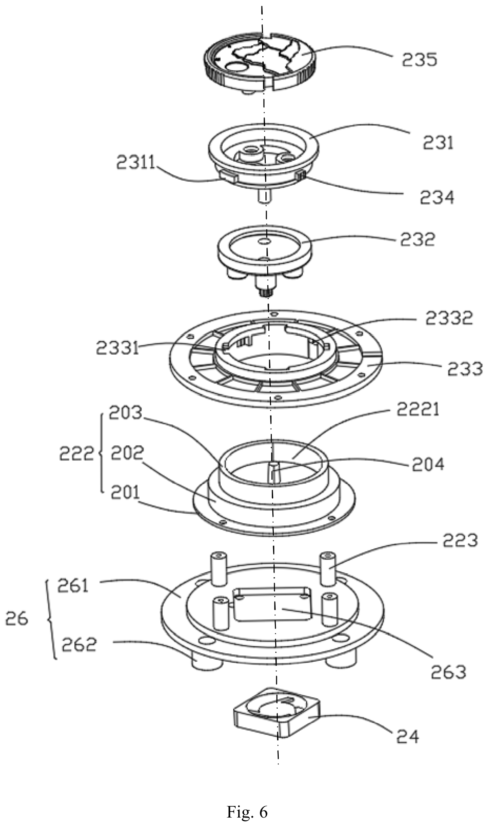

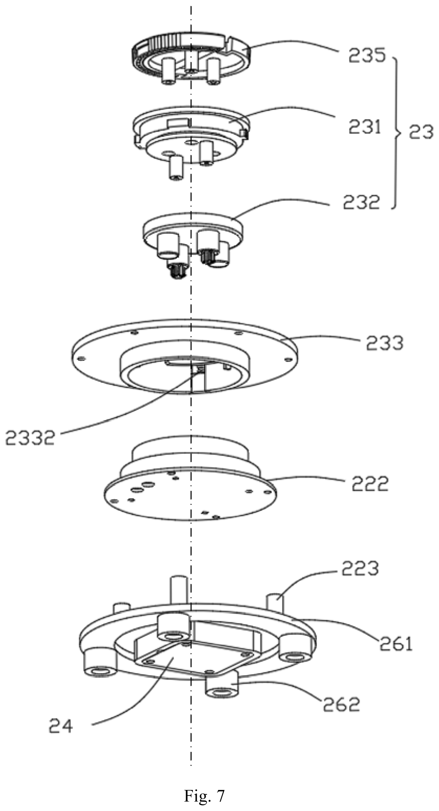

As shown in FIGS. 1-10, provided is a moxibustion therapy pillow including a pillow ring arm 10 and a moxibustion therapy module 20 surrounded by the pillow ring arm 10.

The moxibustion therapy module 20 includes a breathable panel 21, a heating chamber structure 22, and an insertion member 23. The breathable panel 21 is provided with grid holes 211 and an insertion hole 212.

The heating chamber structure includes a chamber cover 221 and a conductive heating element 222 mounted in the chamber cover 221. A chamber opening end of the chamber cover 221 is connected to a bottom surface of the breathable panel 21 and surrounds and covers the grid holes 211 and the insertion hole 212. An air intake 2211 is provided on a bottom wall of the chamber cover 221.

Further, the heating chamber structure includes a heat insulation column 223. The heat insulation column 223 is installed in the chamber cover 221 to support the electric conductive heating element 222 and serves to insulate the electric conductive heating element 222 from the chamber cover 221.

A heating cavity 2221 is formed on the conductive heating element 222 to accommodate the moxa sheet for heating, and the heating cavity 2221 faces the insertion hole 212. It can be understood that the conductive heating element 222 is electrically connected to conductive circuits to supply power to the conductive heating element 222.

The insertion piece 23 is used for placing and removing the moxa sheet. The insertion piece 23 is placeably and removably installed in the insertion hole 212 to accommodate the placed and removed moxa sheet in the heating cavity 2221. The moxa heat emitted from the heated moxa sheet is dispersed through the grid holes.

The moxibustion therapy module 20 further includes a fan 24. The fan 24 is mounted at the position corresponding to the air intake 2211 to blow air into the chamber cover 221, causing the moxa heat to disperse through the grid holes 211.

The moxibustion therapy module 20 further includes a bottom cover 25. A cover opening end of the bottom cover 25 is connected to the bottom surface of the breathable panel 21 and covers the chamber cover 221. The fan 24 is mounted inside the bottom cover 25.

Further, the moxibustion therapy module 20 further includes a fan frame 26 for mounting the fan 24. The fan frame 26 is mounted inside the bottom cover 25, and the fan 24 is mounted on the fan frame 26. It can be understood that the heat insulating column 223 can also be mounted on the fan frame 26 and extend into the chamber cover 221 to support the conductive heating element 222. Preferably, the fan 24 is mounted on the fan frame 26, and an air outlet end of the fan 24 extends into the air intake 2211.

In the present embodiment, the fan frame 26 includes a boss part 261 and a screw sleeve 262 formed on the bottom surface of the boss part 261. The boss part 261 is provided with a through fan mounting hole 263. The heat insulating column 223 is vertically provided on a top surface of the boss part 261. The screw sleeve 262 is formed on the bottom surface of the mounting base 321 and is adjacent to the periphery. The screw sleeve 262 extends through the top surface of the boss part 261 to allow the fan frame 26 to be fixedly connected to the bottom wall of the chamber cover 221. The protruding end of the boss part 261 extends into the air intake 2211, and the bottom end abuts against the bottom wall of the chamber cover 221. Screws are provided to extend through the screw sleeve 262 and lock with the bottom wall of the chamber cover 221, thereby fixing the fan frame 26 to the bottom wall of the chamber cover 221. It can be understood that the screw sleeve 262 can be a support rod fixedly mounted inside the bottom cover 25 to support the boss part 261.

A pillow ring arm 10 can be designed with the outline of a cushion or a bolster, possessing certain elasticity, and can be made by wrapping PU foam cotton with a leather-cloth surface layer. A mounting hole 11 is formed at the center of the pillow ring arm 10 for mounting a moxibustion therapy module 20. Further, step grooves 111 are formed at upper and lower ends of the mounting hole 11 for fitting with the moxibustion therapy module 20 during assembly.

Further, air intake ducts 12 are relatively provided on both sides of the pillow ring arm 10, communicating with the interior of the bottom cover 25 to allow external air to enter the bottom cover 25 through the air intake ducts 12 for the fan 24 to draw in.

Further, a filter sheet 13 is provided at an entrance of the air intake duct 12 for air filtration. The filter sheet 13 is preferably made of a silicone sheet for easy attachment to the entrance of the air intake duct 12.

The breathable panel 21 includes a panel part and a peripheral wall protruding from the bottom surface of the panel part and adjacent to the outer periphery. A peripheral edge is formed between the panel part and the peripheral wall for pressing against the step groove 111. The panel part has one end provided with an insertion hole 212, and another end provided with a light transmitting hole 213 at its center. The insertion hole 212 is used for fitting with the insertion member 23, and the light transmitting hole 213 is used for fitting with the phototherapy device to allow light to pass through, such as blue light, infrared light, etc. The phototherapy device is mounted inside the chamber cover 221. Grid holes 211 are formed on a surface area except for the insertion hole 212 and the light transmitting hole 213, in a grid pattern.

The bottom cover 25 includes a base plate part and a peripheral wall protruding from the top surface of the base plate part and adjacent to the outer periphery. A peripheral edge is formed between the base plate part and the peripheral wall for pressing against the step groove 111. The peripheral wall of the breathable panel 21 is connected to the peripheral wall of the bottom cover 25 to form a chamber that houses the fan 24, the fan frame 26, and the chamber cover 221. It is understandable that gaps corresponding to the air intake ducts 12 are provided on the peripheral walls of the breathable panel 21 or the bottom cover 25 to allow external airflow to enter the chamber.

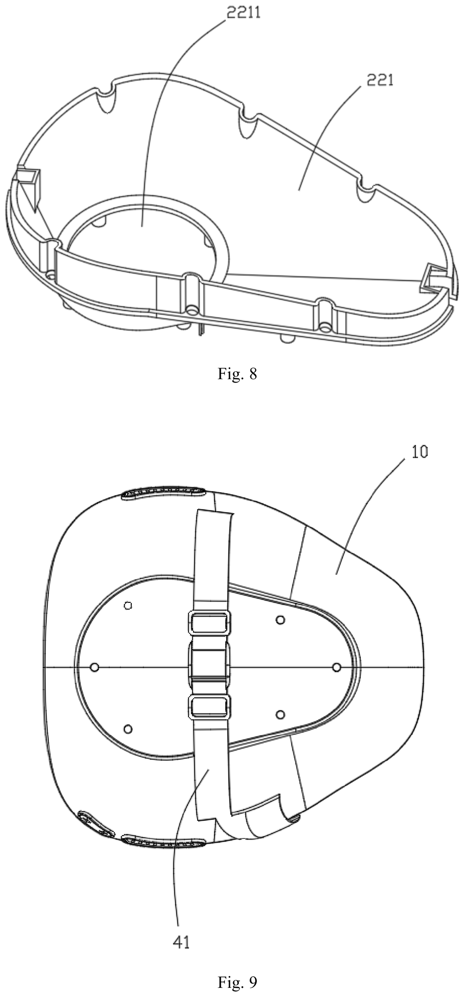

The chamber cover 221 is used to evenly disperse moxa heat from the grid holes 211 of the breathable panel 21. Referring to Fig. 8, the chamber cover 221 is gourd-shaped, with a first end having an air inlet 2211 corresponding to the electric conductive heating element 222. The chamber cover 221 has a second end away from the air inlet 2211. The depth of the inner groove of the chamber cover 221 gradually decreases from the first end to the second end, and the depth of the groove from the air inlet 2211 to its periphery gradually decreases. The depth of the groove from the centerline of the second end to both sides gradually decreases. The inner wall surface of the chamber cover 221 is smoothly transitioned in an arc shape as a whole. Through such a design of the inner wall surface of the chamber cover 221, moxa heat can be evenly dispersed from the entire surface of the grid holes 211. The specific principle is as follows: at the electric conductive heating element 222, the moxa heat temperature is higher, and its distance from the grid holes 211 is large. As it gradually moves away from the electric conductive heating element 222, the temperature gradually decreases, while the bottom surface inside the chamber cover 221 gets closer to the grid holes 211, facilitating the blowout of the airflow. Therefore, through the change in groove depth corresponding to the distance from the electric conductive heating element 222 inside the chamber cover 221, the moxa heat dispersed from the entire grid holes 211 is even, ensuring that the moxibustion area is evenly heated during moxibustion, improving the comfort of moxibustion. It is understandable that the conductive heating element 222 can be inserted between the two ends inside the chamber cover 221, with the groove depth gradually decreasing from the conductive heating element 222 towards both ends.

In the embodiment of the present disclosure, the conductive heating element 222 includes a base plate 201, a heating element 202 formed on the base plate 201, and a chamber tube 203 formed on the heating element 202 to enclose a heating cavity 2221. The base plate 201 is supported on a heat insulating column 223. The heating element 202 is provided with a conductive heating sheet inside. The chamber tube 203 roughly encloses a space corresponding to the size of the moxa sheet for accommodating the moxa sheet. The moxa sheet is heated by the heating element 202 to emit moxa heat. Further, a positioning column 204 is provided at the center of the chamber tube 203 for extending through the moxa sheet to position same.

The insertion member 23 includes a sealing and blocking plate part 231 and heat-resistant prongs 232 provided at a bottom end of the sealing and blocking plate part 231. The sealing and blocking plate part 231 is fixed in cooperation with the insertion hole 212, and the heat-resistant prongs 232 are used for inserting and removing the moxa sheet.

The sealing and blocking plate part 231 can be screwed or snapped into cooperation with the insertion hole 212. In the present embodiment, a snap-fit structure is used for cooperation. Specifically, clasps 2311 are provided on the outer periphery of the sealing and blocking plate part 231, and a snap ring 233 is fixedly provided on a breathable panel 21. A ring hole of the snap ring 233 is aligned with the insertion hole 212, and snap grooves 2331 are provided on the peripheral wall of the ring hole for cooperation with the clasps 2311, allowing the clasps 2311 of the insertion member 23 to pass downward through the corresponding snap grooves 2331 and then rotate the insertion member 23 to fix the insertion member 23 in the insertion hole 212.

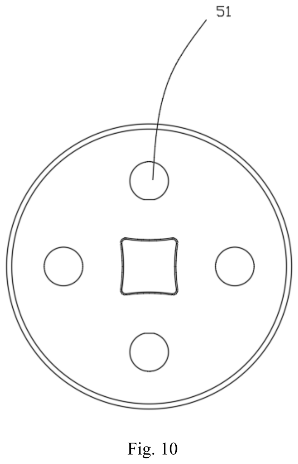

The heat-resistant prongs 232 have excellent heat resistance and thermal insulation properties and are used for grasping the moxa sheet and inserting same into the heating cavity 2221 for heating. Specifically, referring to Fig. 10, an insertion holding hole 51 is provided on the moxa sheet, and the heat-resistant prongs 232 have insertion prongs that are inserted into the insertion holding hole 51 to hold the moxa sheet. Preferably, the insertion member 23 is made of an elastic material, and the insertion prongs elastically press against the insertion holding hole 51 to stably hold the moxa sheet. The elastic material is preferably heat-resistant silicone or a heat-resistant silicone sleeve is sleeved on the insertion prongs, providing heat resistance and excellent thermal insulation properties.

Further, a pressure conduction part 234 is provided on the outer periphery of the insertion member 23 to conduct the heating circuit of the conductive heating element 222 after insertion. Thus, when the insertion member 23 is not inserted, the conductive heating element 222 cannot be activated for heating, eliminating the risk of scalding the user due to no-load heating.

It is understandable that by providing a conductive spring 2332 on the snap ring 233, when the insertion member 23 is not inserted, the conductive spring 2332 pops up to disconnect the heating circuit of the conductive heating element 222. After the insertion member 23 is inserted, the insertion member 23 presses against the conductive spring 2332 to connect the heating circuit of the conductive heating element 222, allowing the heating of the conductive heating element 222 to be started by turning on the heating switch.

Further, a handle cover 235 is provided at the top end of the insertion member 23 to facilitate rotating the insertion member 23 through the handle cover 235 for easy snapping into the insertion hole 212 of the breathable panel 21. It is understandable that decorative graphics, such as LOGOs, can be formed on the top surface of the handle cover 235.

It is understandable that the moxibustion therapy pillow includes a circuit board and a power supply, which can be inserted inside the bottom cover 25 and are electrically connected to the fan 24, the heating element 202, and the phototherapy device for supplying power and electrical control to the fan 24, the heating element 202, and the phototherapy device.

It is understandable that the moxibustion therapy pillow includes a controller, which is electrically connected to the circuit board through wires for controlling the on/off and gear adjustment of the fan 24, the heating element 202, and the phototherapy device. Further, a cloth bag is formed on the outer periphery of the pillow ring arm 10 for accommodating the controller.

It is understandable that the moxibustion therapy pillow includes an elastic band 41 mounted on the bottom surface of the bottom cover 25 for elastically binding the moxibustion therapy pillow to external objects, such as household chairs or car seats, using the moxibustion therapy pillow as a backrest.

It is understandable that to further adjust the uniformity of heat dissipation, the inclination angle of the grid holes 211 can be adjusted.

It is understandable that the grid holes 211 can also be of other hole types, if moxa heat can be dispersed from the breathable panel 21.

When using the moxibustion therapy pillow of the present disclosure, the moxa sheet is inserted and retrieved using the insertion member 23 and mounted in the insertion hole 212. The moxa sheet is accommodated in the heating cavity 2221, and then the conductive heating element 222 is activated. The moxa sheet is heated to emit moxa heat, which is dispersed from the cavity opening of the heating cavity 2221 into the chamber cover 221. The fan 24 draws in air from inside the bottom cover 25 and blows it into the chamber cover 221. The gas inside the bottom cover 25 flows in through the air intake ducts 12 on both sides of the pillow ring arm 10. The moxa heat inside the chamber cover 221 is dispersed from the grid holes 211. Through the structural design of the chamber cover 221, the moxa heat can be evenly dispersed from the grid holes 211, forming an airflow path that enters from both sides of the pillow ring arm 10 and disperses from the grid holes 211 of the breathable panel 21.

The moxibustion therapy pillow of the present disclosure heats the moxa sheet using a conductive heating element, obviating the need for open flame moxibustion and ensuring safe use. The moxa sheet is heated inside the heating chamber structure by inserting and removing it using the insertion member, and the moxa heat is dispersed from the grid holes of the breathable panel. The moxa sheet is stably mounted on the insertion member during heating, insertion, or removal, making it easy to mount and retrieve and providing good heat stability. The grid holes can cover a large area on the breathable panel, enabling large-area moxa heat dispersion and facilitating large-area moxibustion on local areas of the body. The moxibustion pillow is convenient for daily use. Further, through the structural design of the chamber cover, the dispersed moxa heat is uniformly applied to the user's body surface, improving the comfort and therapeutic effect of moxibustion.

Any combination of the various different embodiments of the present disclosure, provided that it does not depart from the inventive concept of the present disclosure, shall be deemed as the content disclosed by the present disclosure; various simple modifications to the technical solution, as well as any combination of the different embodiments that does not depart from the inventive concept of the present disclosure, within the scope of the technical concept of the present disclosure, shall all fall within the scope of protection of the present disclosure.

## Claims

1. A moxibustion therapy pillow comprising a pillow ring arm (10) and a moxibustion therapy module (20) surrounded by the pillow ring arm,
the moxibustion therapy module (20) comprising a breathable panel (21), a heating chamber structure (22), and an insertion piece (23),
wherein:
the breathable panel (21) is provided with an insertion hole (212),
the heating chamber structure (22) comprises a chamber cover (221) and an electric conductive heating element (222) installed in the chamber cover (221), a chamber opening end of the chamber cover (221) being connected to a bottom surface of the breathable panel (21),
the insertion piece (23) is configured to be placeably and removably installed in the insertion hole (212) for delivering a moxa sheet to the electric conductive heating element (222) for heating, the moxa heat emitted from the heated moxa sheet being configured to be dispersed through the breathable panel (21).

2. The moxibustion therapy pillow according to claim 1, wherein
an air intake (2211) is provided on a bottom wall of the chamber cover (221),
the moxibustion therapy module (20) comprises a fan (24), the fan (24) being configured to be mounted at a position of the air intake (2211) in order to blow air into the chamber cover (221), so that the moxa heat is dispersed through the breathable panel (21).

3. The moxibustion therapy pillow according to claim 2, wherein a heat insulating column (223) mounted in the chamber cover (221), the heat insulating column (223) being configured to support the conductive heating element (222).

4. The moxibustion therapy pillow according to claim 2,
wherein
the moxibustion therapy module (20) comprises a bottom cover (25), the bottom cover (25) being connected to the bottom surface of the breathable panel (21) and covering the chamber cover (221),
and wherein the fan (24) is mounted inside the bottom cover (25).

5. The moxibustion therapy pillow according to claim 4, wherein an air intake duct (12) is provided on the pillow ring arm (10) to communicate with the interior of the bottom cover (25).

6. The moxibustion therapy pillow according to claim 4, wherein a power supply and a circuit board are mounted inside the bottom cover (25).

7. The moxibustion therapy pillow according to claim 1, wherein the breathable panel (21) comprises a panel part, on which a light transmitting hole (213) is formed, and the light transmitting hole (213) is used in conjunction with a phototherapy device to allow light to pass through.

8. The moxibustion therapy pillow according to claim 1, wherein the insertion member (23) comprises a sealing and blocking plate part (231) and heat-resistant prongs (232) formed at a bottom end of the sealing and blocking plate part (231), the sealing and blocking plate part (231) being fitted with the insertion hole (212) for fixation, and the heat-resistant prongs (232) are configured to insert and remove the moxa sheet.

9. The moxibustion therapy pillow according to claim 8, wherein a pressure conduction part (234) is provided on the sealing and blocking plate part (231) to conduct the heating circuit of the conductive heating element (222) when the insertion member (23) is inserted.

10. The moxibustion therapy pillow according to claim 1, comprising an elastic band (41), both ends of which being connected to the pillow ring arm (10) for elastically binding the moxibustion therapy pillow to an external object.
